Europäisches Patentamt

**European Patent Office**

Office européen des brevets

(11) Publication number: **0 069 600**
**A2**

# EUROPEAN PATENT APPLICATION

(21) Application number: 82303582.9

(22) Date of filing: 08.07.82

(51) Int. Cl.³: **A 61 K 31/485**
**A 61 K 9/08**

(30) Priority: 10.07.81 GB 8121315

(43) Date of publication of application:
12.01.83 Bulletin 83/2

(84) Designated Contracting States:
BE CH DE FR IT LI LU NL SE

(71) Applicant: RECKITT AND COLMAN PRODUCTS
LIMITED
P.O. Box 26 1-17, Burlington Lane
London W4 2RW(GB)

(72) Inventor: Todd, Richard Sydney
37 Burton Road
Cottingham North Humberside(GB)

(74) Representative: Hardisty, David Robert et al,
BOULT, WADE & TENNANT 27 Furnival street
London EC4A IPQ(GB)

(54) Pharmaceutical compositions.

(57) A pharmaceutical composition for sublingual administration comprising buprenorphine or a non-toxic salt thereof
dissolved in 20-30% v/v aqueous ethanol buffered to between pH 4.5 to 5.5 with 0.05 to 0.2 molar concentration of a
buffering agent selected from citric acid/disodium hydrogen
phosphate, sodium citrate/hydrochloric acid, lactic acid/
disodium hydrogen phosphate, lactic acid/sodium lactate,
sodium citrate/citric acid and sodium acetate/acetic acid, the
concentration of buprenorphine being between 0.8 and 10
mg/ml of the composition. Buprenorphine is a potent
antagonist analgesic with good bioavailability following
sublingual administration, useful in the relief of moderate to
severe pain and also in the treatment of natcotic addiction.
The compositions enable higher doses of buprenorphine to
be administered sublingually.

EP 0 069 600 A2

Croydon Printing Company Ltd.

- 1 -

## Pharmaceutical Compositions

This invention relates to pharmaceutical compositions and more particularly to compositions containing buprenorphine.

Buprenorphine (International Non-proprietary Name for N-cyclopropylmethyl-7α-(1-(S)-hydroxy-1,2,2-trimethyl-propyl)6,14-endoethano-6,7,8,14-tetrahydronororipavine) has been shown in clinical trials to be a potent antagonist analgesic lacking the psychotomimetic effects found with other antagonist analgesics. Buprenorphine effectively relieves moderate to severe pain in doses of 0.15mg or more administered either parenterally or sublingually. The optimum therapeutic range for single doses is 0.3mg - 0.6mg by injection and 0.2 - 0.4mg for sublingual tablets. The drug has also been shown to have utility in the treatment of narcotic addiction. In patients suffering from a chronic condition such as intractable (cancer) pain or requiring treatment for narcotic addiction multi-dosing and/or higher dosage levels are required. To avoid patient discomfort from too frequent injections the sublingual route is indicated, since the drug is poorly absorbed orally. With a tablet presentation there is a physical limit to the number of tablets which can be retained under the tongue at any one time and as a result there can be considerable patient variability as to the blood levels of the drug and hence its biological effects.

We have carried out investigations in an attempt to produce a liquid composition containing buprenorphine which can be administered sublingually. Our studies have shown that the maximum volume of liquid that a patient can hold sublingually for a reasonable amount of time without swallowing is 1ml and that a smaller volume is preferred say 0.5ml.

We have found it very difficult to prepare stable aqueous solutions of adequate concentration for sublingual administration. We have discovered in patients that using solutions buffered at pH's 4, 5 and 6 respectively with citric acid/disodium hydrogen phosphate buffer that following administration of a 400µg/0.5ml dose of buprenorphine hydrochloride by the subligual route that the degree of uptake of the drug was greater at the two higher pH's.

We have now developed stable liquid compositions containing buprenorphine at a high concentration suitable for sublingual administration.

According to the present invention there is provided a pharmaceutical composition for sublingual administration comprising buprenorphine or a non-toxic salt thereof dissolved in 20-30% v/v aqueous ethanol buffered to between pH 4.5 to 5.5 with 0.05 to 0.2 molar concentration of a buffering agent selected from citric acid/disodium hydrogen phosphate, sodium citrate/hydrochloric acid, lactic acid/disodium hydrogen phosphate, lactic acid/sodium lactate, sodium citrate/citric acid and sodium acetate/acetic acid, the concentration of buprenorphine being between 0.8 and 10 mg/ml of the composition.

A preferred salt is the hydrochloride. Preferably the molar concentration of the buffering agent is between 0.1 and 0.15.

The sodium citrate in the sodium citrate/hydrochloric acid or sodium citrate/citric acid buffering agent can be the trisodium or disodium salt according to the pH required.

By means of the present invention it is possible to administer doses of between 0.4 to 5.0mg of buprenorphine in 0.5ml of solution sublingually. Patients requiring such high doses will normally be in an institution where self administration will not be the norm. The composition will normally be administered by a doctor or medically trained personnel using for example a suitable dispenser. If the dispenser is calibrated a lesser metered amount can be administered if a lesser dosage is required particularly in the treatment of intractable pain where is it desirable to keep doses low initially to give adequate pain relief but with the opportunity of increasing the dosage if and when it becomes necessary. The composition may also be presented in a unit dosage form in a form-fill-seal plastics container with a nominal volume of 0.5 to 1.0ml, suitably the plastics being polythene or polypropylene.

- 4 -

An example of such a plastics container is Minims (Registered Trade Mark, Smith & Nephew).

The invention is illustrated by the following Examples:-

Example 1

Preparation of 10mg/ml buprenorphine solution

10ml of a 10mg/ml buprenorphine solution in a pH5 mixture of a ~30% v/v aqueous ethanol: citric acid/disodium hydrogen phosphate buffer was prepared as follows:

1. The buffer was prepared by mixing 3.8ml 0.1 M citric acid (21g/l of monohydrate) and 3.2ml 0.2 M disodium hydrogen phosphate (35.6g/l of dihydrate).

2. 3.0ml 95% v/v ethanol was added to the buffer increasing the pH from ~4.6 to ~5.0.

3. 108mg buprenorphine hydrochloride was added with stirring until dissolved.

Examples 2 to 13

The formulation of Example 1 was varied by employing differing volumes and molar strengths of the citric acid and disodium hydrogen phosphate solutions, and differing volumes of the 95% v/v ethanol, and in some cases adding additional water before dissolving the buprenorphine hydrochloride. In the Table, Vol = volume, M = Molarity and the pH is that of buffer plus ethanol.

Table

| Ex. | Citric acid Vol. ml | Citric acid Mol. M | Phosphate Vol. ml | Phosphate Mol. M | Addit. Water ml | Ethanol Vol. ml | Overall Ethanol % v/v conc. | pH |
|---|---|---|---|---|---|---|---|---|
| 2 | 4.2 | 0.1 | 2.8 | 0.2 | 0.9 | 2.1 | 20.0 | 4.5 |
| 3 | 4.25 | 0.1 | 2.75 | 0.2 | - | 3.0 | 28.5 | 4.5 |
| 4 | 3.6 | 0.1 | 3.4 | 0.2 | 0.9 | 2.1 | 20.0 | 5.0 |
| 5 | 3.4 | 0.1 | 3.6 | 0.2 | - | 3.0 | 28.5 | 5.5 |
| 6 | 4.2 | 0.05 | 2.8 | 0.1 | 0.9 | 2.1 | 20.0 | 4.5 |
| 7 | 3.7 | 0.05 | 3.3 | 0.1 | 0.9 | 2.1 | 20.0 | 5.0 |
| 8 | 3.8 | 0.05 | 3.2 | 0.1 | - | 3.0 | 28.5 | 5.0 |
| 9 | 3.5 | 0.05 | 3.5 | 0.1 | - | 3.0 | 28.5 | 5.5 |
| 10 | 4.1 | 0.2 | 2.9 | 0.4 | 0.9 | 2.1 | 20.0 | 4.5 |
| 11 | 3.6 | 0.2 | 3.4 | 0.4 | 0.9 | 2.1 | 20.0 | 5.0 |
| 12 | 3.75 | 0.2 | 3.25 | 0.4 | - | 3.0 | 28.5 | 5.0 |
| 13 | 3.3 | 0.2 | 3.7 | 0.4 | - | 3.0 | 28.5 | 5.5 |

## Examples 14-18

The method of Example 1 was varied by employing a similar volume of differing buffers as follows:

## Example 14

Sodium citrate/hydrochloric acid: mixed 5.6 ml 0.2M disodium citrate (42.0g citric acid monohydrate +400 ml 1 N sodium hydroxide/1) and 1.4 ml 0.2M hydrochloric acid. On adding the ethanol the pH increased from ~4.6 to ~5.0.

## Example 15

Lactic acid/disodium hydrogen phosphate: mixed 4.0 ml 0.2M lactic acid (20.4g/1 of 88% lactic acid) and 3.0 ml 0.2M disodium hydrogen phosphate (35.6g/1 of dihydrate). On adding the ethanol the pH increased from 4.5 to ~5.0.

Example 16

Lactic acid/sodium lactate: mixed 1.3 ml 0.2M lactic acid (20.4g/1 of 88% lactic acid) and 5.7 ml 0.2M sodium lactate (20.4g of 88% lactic acid +200ml 1 N sodium hydroxide/1). On adding the ethanol the pH increased from 4.5 to ~5.0.

Example 17

Sodium citrate/citric acid: mixed 4.15 ml 0.2M sodium citrate (58.8g/1 trisodium citrate dihydrate) and 2.85ml 0.2M citric acid (42.0g/1 citric acid monohydrate). On adding the ethanol the pH increased from ~4.6 to ~5.0.

Example 18

Sodium acetate/acetic acid: mixed 3.5 ml 0.2M sodium acetate (16.4g/1) and 3.5 ml 0.2M acetic acid (12.0g/1). On adding the ethanol the pH increased from ~4.6 to ~5.0.

Claims

1. A pharmaceutical composition for sublingual administration comprising buprenorphine or a non-toxic salt thereof dissolved in 20-30% v/v aqueous ethanol buffered to between pH 4.5 to 5.5 with 0.05 to 0.2 molar concentration of a buffering agent selected from citric acid/disodium hydrogen phosphate, sodium citrate/ hydrochloric acid, lactic acid/disodium hydrogen phosphate, lactic acid/sodium lactate, sodium citrate/citric acid and sodium acetate/acetic acid, the concentration of buprenorphine being between 0.8 and 10 mg/ml of the composition.

2. A pharmaceutical composition as claimed in claim 1 wherein the buprenorphine salt is the hydrochloride.

3. A pharmaceutical composition as claimed in claim 1 or claim 2 wherein the molar concentration of the buffering agent is between 0.1 and 0.15.

4. A pharmaceutical composition as claimed in claim 2 wherein the buffering agent is citric acid/disodium hydrogen phosphate.

DRH/EA654